(19) Europäisches Patentamt
European Patent Office
Office européen des brevets

(11) EP 1 229 890 B1

(12) **FASCICULE DE BREVET EUROPEEN**

(45) Date de publication et mention de la délivrance du brevet:
**15.09.2004 Bulletin 2004/38**

(21) Numéro de dépôt: **01976384.6**

(22) Date de dépôt: **09.10.2001**

(51) Int Cl.[7]: **A61K 7/06**, A61K 7/48, A61K 7/13, A61K 7/42

(86) Numéro de dépôt international:
**PCT/FR2001/003109**

(87) Numéro de publication internationale:
**WO 2002/030371 (18.04.2002 Gazette 2002/16)**

(54) **COMPOSITION TINCTORIALE FAVORISANT LA PIGMENTATION NATURELLE, PROCEDE D'OBTENTION ET UTILISATION POUR LA COLORATION DE LA PEAU ET/OU DES FIBRES KERATINIQUES**

FÄRBEMITTEL ZUR FÖRDERUNG DER NATÜRLICHEN PIGMENTIERUNG, VERFAHREN ZUR HERSTELLUNG UND VERWENDUNG ZUM FÄRBEN DER HAUT UND/ODER DER KERATINISCHEN FASERN

DYEING COMPOSITION PROMOTING NATURAL PIGMENTATION, METHOD FOR OBTAINING SAME AND USE FOR COLOURING THE SKIN AND/OR KERATINOUS FIBRES

(84) Etats contractants désignés:
**AT BE CH CY DE DK ES FI FR GB GR IE IT LI LU MC NL PT SE TR**

(30) Priorité: **09.10.2000 FR 0012897**

(43) Date de publication de la demande:
**14.08.2002 Bulletin 2002/33**

(73) Titulaire: **L'OREAL**
**75008 Paris (FR)**

(72) Inventeur: **PRUCHE, Françis**
**F-60300 SENLIS (FR)**

(74) Mandataire: **Catherine, Alain**
**Cabinet Harlé & Phélip**
**7, rue de Madrid**
**75008 Paris (FR)**

(56) Documents cités:
**GB-A- 2 307 175**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 229 890 B1

## Description

La présente invention concerne d'une manière générale une composition tinctoriale, en particulier pour la coloration de la peau et/ou des matières kératiniques qui favorise la pigmentation naturelle de la peau et/ou des fibres kératiniques.

La couleur des cheveux et de la peau humaine est fonction de différents facteurs et notamment des saisons de l'année, de la race, du sexe et de l'âge. Elle est principalement déterminée par la concentration de mélanine produite par les mélanocytes. Les mélanocytes sont les cellules spécialisées qui par l'intermédiaire d'organelles particuliers, les mélanosomes, synthétisent la mélanine.

La synthèse de la mélanine ou mélanogénèse est particulièrement complexe et fait intervenir schématiquement les principales étapes suivantes :

Tyrosine → Dopa → Dopaquinone → Dopachrome → Mélanine

La tyrosinase (EC 1.14.18.1) est l'enzyme responsable de la catalyse de deux réactions successives : l'hydroxylation d'un monophénol (exemple la tyrosine) en un o-diphénol (exemple la L-DOPA) et l'oxydation de cet o-diphénol en o-quinone et une troisième réaction qui est l'oxydation du 5,6 Dihydroxyindole. Deux autres enzymes jouent un rôle : la TRP2 (Dopachrome tautomérase) et la TRP1 (DHIC oxydase). Chez les plantes, il existe des enzymes différentes pour ces deux réactions. L'oxydation des polyphénols se réalise par des polyphenols oxydases de deux types : les laccases (EC 1.10.3.2) qui oxydent les p-diphénols et les catéchol oxydases (EC 1.10.3.1) qui oxydent les o-diphénols. Le site actif de ces enzymes contient du cuivre. Les réactions après la formation de dopaquinones peut se réaliser d'une façon non-enzymatique et en particulier les réactions avec la L-cystéine responsables de la formation de phéomélanines (qui donnent une coloration jaune/marron à rouge).

Chez les plantes, il existe de nombreux substrats de la catécholase comme la catéchine, l'acide chlorogénique, le catéchol. Les réactions oxygène dépendantes sont responsables du « brunissement enzymatique » des fruits. Cette enzyme est importante aussi dans les réactions de fermentation de certaines plantes ou fruits. Par exemple, dans la fabrication des thés colorés, la catécholase est responsable de la formation des pigments.

Chez l'homme, la teneur en mélanine varie d'une population à l'autre et la quantité de tyrosinase et en ARNm de celle-ci ne varient pas significativement entre une peau noire et une peau blanche. C'est donc au niveau de la régulation de l'activité tyrosinase qu'une intervention est souhaitable. C'est la raison pour laquelle les produits ayant un effet dépigmentant ont pour cible cette enzyme (exemple hydroquinone et l'acide Kojique) ou en intervenant au niveau des quinones formées (par exemple des antioxydants comme l'acide ascorbique). Pour la modulation de la pigmentation de nombreuses solutions ont été proposées mais font intervenir des hormones (alpha MSH) ou des molécules agissant sur des seconds messagers (type AMPc). Le mécanisme de la formation de mélanine est très complexe et les mécanismes précis ne sont pas encore élucidés.

La recherche de composés pouvant augmenter la pigmentation de la peau et du cheveu est une préoccupation de la dermatologie et de la cosmétique.

A cet égard, il a été proposé de nombreuses solutions dans le domaine de la coloration artificielle, par apport de colorants exogènes sensés donner à la peau et/ou les cheveux une coloration le plus proche de ce qu'elle est naturellement ou dans le domaine de la coloration naturelle par stimulation des voies naturelles de pigmentation.

D'excellents résultats sont certes obtenus par les solutions proposées dans l'art antérieur, mais il n'en demeure pas moins que la stimulation de la pigmentation de la peau et/ou des cheveux par la voie naturelle reste la voie idéale de pigmentation.

A cet égard, il a été proposé dans les documents WO-A-9517161, WO-9511003, WO-A-9501773, WO-A-9404674, WO-A-9404122, EP-A-585018, WO-A-9310804, WO-A-9220322 ou WO-A-9107945 des solutions aussi variées que des compositions contenant un inhibiteur de phosphodiestérases, l'utilisation de prostaglandine, de fragments d'ADN, de dérivés de la tyrosine ou encore d'extraits de plantes.

Souvent, les composés utilisés présentent des effets secondaires non négligeables ou sont des mélanges complexes qui ne présentent pas de spécificité.

Il a d'autre part été suggéré par Rushton et collaborateurs (Rushton, D.H., et coll., Clin. Exp. Dermatol., 1989, 14(1), 40-46) que le Minoxidil ou 2,4-diamino-6-piperidino pyrimidine 3-oxyde, pouvait avoir un effet stimulateur de la pigmentation du velus chez des hommes chauves traités par ce composé.

Le « Minoxidil » est connu pour ses effets anti-hypertenseur et pour sa capacité à favoriser la pousse des cheveux. Ces propriétés sont décrites dans US-4.596.812

Le Minoxidil, s'il reste le composé de référence dans le domaine de la pousse des cheveux, présente des effets secondaires non négligeables . qui rendent son utilisation délicate.

La demande WO-A-9220321 décrit une crème favorisant le brunissement de peaux claires lors d'une exposition au soleil ou à des rayons UVB, dont la composition comprend un écran solaire, un milieu physiologiquement

acceptable et une pseudocatalase. La pseudocatalase est un complexe de coordination d'un métal de transition dont le métal est Cu(I), Fe(II) ou Mn(II) et le ligand du bicarbonate. Par pseudocatalase, on entend un composé physiologiquement acceptable qui catalyse la dismutation de $H_2O_2$ in vivo de manière analogue à une catalase.

Pour le traitement de la dépigmentation de la peau liée à des blocages de la transformation de la tyrosine en mélanine, comme par exemple le vitiligo, la demande WO-9220354 décrit une composition contenant, dans un milieu physiologiquement acceptable, une pseudocatalase.

L'article de K. Schallreuter («Pseudocatalase is a bis-manganese III-EDTA-(HCO3)2 complex activated by UVB or natural sun»; J Investing Dermator Symp Proc 1999 Sep; 451°; 91-6) mentionne l'utilisation d'un mélange d'hydrogénocarbonate de sodium et de manganèse ayant une activité pseudocatalase pour le traitement du vitiligo. Cependant, il n'y a pas, dans l'ensemble de ces documents, d'indication concernant la coloration.

Dans le domaine de la coloration capillaire, le brevet européen EP-A-6.210.029 décrit une composition comprenant du chlorite de sodium, un sel hydrosoluble de Fe, Mn ou Cu, ou un chélate de ce sel et un précurseur de colorant d'oxydation.

La coloration des cheveux nécessite l'emploi de combinaisons $H_2O_2$- ammonium ou amine.

Il serait donc souhaitable de disposer de compositions capables de compenser une déficience en activité enzymatique ou de se substituer aux cellules productrices de pigments (mélanocytes).

Il serait également souhaitable de disposer de telles compositions qui permettraient également une coloration de la peau et/ou des matières kératiniques par apport de colorants exogènes.

De telles composition seraient particulièrement intéressantes pour le formulation de compositions de bronzage car elles permettraient à la fois d'obtenir un bronzage artificiel et une activation du bronzage naturel.

Dans le cas de compositions pour la teinture des cheveux, ces compositions pourraient permettre des délais prolongés entre deux colorations, l'activation de la pigmentation naturelle des cheveux réduisant la visibilité de la perte de coloration à la racine des cheveux lors de leur pousse.

La demanderesse a trouvé de façon surprenante qu'un système catalytique comprenant l'association d'un sel ou oxyde de Mn(II) et/ou Zn(II) et d'un hydrogénocarbonate alcalin ou alcalino-terreux, permet de formuler des compositions ayant l'activité recherchée.

La présente invention a donc pour objet une composition pour la coloration de la peau et/ou des matières kératiniques par augmentation de l'activité enzymatique.

La présente invention a aussi pour objet une composition telle que définie ci-dessus et dont l'effet colorant est également obtenu par apport de colorants exogènes.

La présente invention a encore pour objet un procédé d'obtention d'une composition tinctoriale telle que définie ci-dessus.

La présente invention concerne en outre un procédé de coloration de la peau et/ou des fibres kératiniques utilisant une composition telle que définie ci-dessus.

Enfin, la présente invention concerne des conditionnements et formes galéniques de la composition tinctoriale ou de constituants de la composition tinctoriale selon l'invention.

La composition pour la coloration de la peau et/ou des fibres kératiniques selon l'invention comprend, dans un milieu physiologiquement acceptable, une quantité efficace d'au moins une enzyme ayant une activité de pigmentation et une quantité efficace d'un sytème catalytique comprenant une premier constituant choisi parmi les sels et oxydes de Mn(II) et/ou Zn(II) et leurs mélanges et un second constituant choisi parmi les hydrogénocarbonates alcalins, les hydrogénorcabonates alcalino-terreux et leurs mélanges, les proportions du premier constituant et du second constituant étant telles que :

$$\frac{[Mn(II)]}{[HCO_3]} \leq 1 \textit{ avec } [\mathrm{Mn(II)}] \neq 0$$

$$\frac{[Zn(II)]}{[HCO_3]} \leq 1 \textit{ avec } [\mathrm{Zn(II)}] \neq 0$$

$$\frac{[Mn(II) + \mathrm{Zn(II)}]}{[HCO_3]} \leq 1 \textit{ avec } [\mathrm{Mn(II)}] \text{ et } [\mathrm{Zn(II)}] \neq 0$$

où [Mn(II)], [Zn(II)] et $[HCO_3]$ représentent respectivement les concentrations molaires en Mn(II), Zn(II) et $HCO_3$ dans la composition.

Dans une autre réalisation de l'invention, la composition comprend en outre une quantité efficace d'au moins

un précurseur de colorant d'oxydation choisi parmi les composés comportant au moins un cycle aromatique ayant au moins deux groupes hydroxyles (OH) portés par deux atomes de carbone consécutifs du cycle aromatique.

Le système catalytique selon l'invention agit alors également comme catalyseur dans l'oxydation du précurseur pour obtenir la forme colorée.

Dans encore une autre réalisation, la composition comprend également une quantité efficace d'au moins un acide aminé comportant au moins un groupe thiol.

Généralement, le rapport $\frac{[Mn(II)]}{[HCO_3]}$ varie de $10^{-5}$ à $10^{-1}$, de préférence de $10^{-3}$ à $10^{-2}$ et est typiquement de l'ordre de $5.10^{-3}$.

Dans le cas de Zn(II), le rapport $\frac{[Zn(II)]}{[HCO_3]}$ est en général d'un ordre de 10 à 100 fois supérieur au rapport dans le cas de Mn(II).

Typiquement, ce rapport est de $10^{-4}$ ou plus, de préférence $10^{-3}$ ou plus, et de préférence de l'ordre de 5.10-1.

Dans le cas d'un mélange de Mn(II) et Zn(II), le rapport varie généralement de $10^{-5}$ à $10^{-1}$, de préférence $10^{-3}$ à $10^{-2}$, ce rapport étant choisi plus élevé lorsque la proportion de Zn(II) dans le mélange s'accroît.

Généralement, la concentration molaire en Mn(II), Zn(II), ou Mn(II) + Zn(II) dans la composition finale varie de $10^{-3}$ à 10 mM/l, de préférence de $10^{-2}$ à 1 mM/l.

Lorsqu'on utilise seulement un ou plusieurs sels ou oxydes de Mn(II), la concentration molaire en Mn(II) dans la composition finale est typiquement de $10^{-3}$ à $10^{-1}$ mM/l, de préférence $10^{-2}$ à $10^{-1}$ mM/l.

De préférence, lorsqu'on utilise uniquement un ou plusieurs sels ou oxydes de Zn(II), la concentration en Zn (II) dans la composition finale est de $5.10^{-2}$ à 10 mM/l, mieux de $5.10^{-1}$ à 1 mM/l.

Parmi les sels de Mn(II) et Zn(II) convenant pour la présente invention, on peut citer les chlorure, fluorure, iodure, sulfate, phosphate, nitrate et perchlorate, les sels d'acides carboxyliques et leurs mélanges.

A titre d'exemple, on peut citer le chlorure de manganèse, le carbonate de manganèse (par exemple rhodochrosite), le difluorure de Mn(II), l'acétate de Mn(II) tétrahydraté, le lactate de Mn(II) trihydraté, le phosphate de Mn (II), le perchlorate de Mn(II) tétrahydraté et le sulfate de Mn(II) monohydraté.

Les sels particulièrement préférés sont $MnCl_2$ et $ZnCl_2$.

Les sels d'acides carboxyliques incluent également des sels d'acides carboxyliques hydroxylés tels que le gluconate.

Parmi les hydrogénocarbonates alcalins et alcalino-terreux, on peut citer les hydrogénocarbonates de Na, K, Mg, Ca et leurs mélanges, préférentiellement l'hydrogénocarbonate de Na.

Comme indiqué précédemment, le système catalytique chimique selon l'invention constitue une pseudo-oxydase en ce qu'il oxyde les polyphénols, en présence d'oxygène, comme le ferait un catalyseur enzymatique naturel ayant une activité polyphénoloxydase.

Par contre, le système catalytique selon l'invention n'a pas d'activité pseudocatalase en ce sens qu'il ne provoque pas la dismutation du peroxyde d'hyrogène à 0,3% en poids (soit 1 volume d'oxygène).

En outre, l'activité pseudo-oxydase est liée à l'emploi du système catalytique selon l'invention. Ainsi, chacun des constituants du système catalytique pris séparément n'a pas d'activité pseudo-oxydase. De même que le remplacement du sel de Mn(II) ou Zn(II) par un autre sel, Fe, Cu ou même Mn(II) ne conduit pas à un système catalytique ayant une activité pseudo-oxydase.

Comme cela sera mis en évidence ci-après, le système catalytique accroît l'activité propigmentation de l'enzyme ou des enzymes présentes dans la composition.

L'enzyme ou les enzymes présentes dans les compositions selon l'invention peuvent être toutes enzymes présentant une activité propigmentation.

L'activité propigmentation peut se définir comme l'activité enzymatique qui catalyse l'oxydation d'un substrat pour conduire à la formation de pigments.

Les enzymes peuvent notamment être choisis parmi les pyranose oxydases, les glucose oxydases, les glycérol oxydases, les lactate oxydases, les pyruvate oxydases, les uricases, les choline oxydases, les sarcosine oxydases, les bilirubines oxydases, les laccases, les tyrosinases, les péroxydases, les catalases, les superoxydesdimutases et leurs mélanges, ou parmi des extraits végétaux ou animaux contenant des enzymes précitées, en présence éventuelle d'un donneur (ou substrat) nécessaire au fonctionnement desdites enzymes tel que par exemple la L-tyrosine ou la L-DOPA.

Les enzymes utilisées selon l'invention peuvent être d'origine animale, microbiologique (bactérienne, fongique ou virale) ou synthétique (obtenue par synthèse chimique ou biotechnologique).

La ou les enzymes peuvent être utilisées sous forme cristalline pure ou sous une forme diluée dans un diluant inerte pour ladite enzyme.

A titre d'exemple d'uricases, on peut notamment citer l'uricase extraite de foie de sanglier, l'uricase d'Arthrobacter globiformis, ainsi que l'uricase d'Aspergillus flavus.

A titre d'exemple de sources de choline oxydase, on peut notamment citer le foie de rat, les bactéries telles que Arthrobacter globiformis, Achromobacter cholinophagum ou Alcaligenes, et les champignons tels que Cylindrocarpon didynum.

A titre d'exemple de sources de sarcosine oxydase, on peut notamment citer les bactéries telles que Arthrobacter et en particulier Arthrobacter ureafaciens et Arthrobacter globiformis, Streptomyces, Bacillus, Pseudomonas, Corynebacterium ou Alcaligenes tels que par exemple Alcaligenes denitrificans, et les champignons tels que Cylindrocarpon didynum.

A titre d'exemple de sources de bilirubine oxydase, on peut notamment citer la muqueuse intestinale et le foie de rat, les bactéries telles que Myrothecium verucania, Myrothecium cinctum, et Myrothecium roridum.

Parmi les laccases d'origine végétale utilisables selon l'invention, on peut citer les laccases produites par des végétaux effectuant la synthèse chlorophylienne telles que celles indiquées dans la demande de brevet FR-A-2.694.018.

On peut notamment citer les laccases extraites d'Anacardiacées, de Podocarpacées, de Rosmarinus off., de Solanum tuberosum, dIris sp., de Coffea sp., de Daucus carrota, de Vinca minor, de Persea americana, de Catharenthus roseus, de Musa sp., de Malus pumila, de Gingko biloba, et de Monotropa hypopithys (sucepin).

Parmi les laccases d'origine microbienne (notamment fongique), ou obtenues par biotechnologie utilisables selon l'invention, on peut citer les laccases de Polyporus versicolor, de Rhizoctonia praticola et de rhus vernicifera telles que décrites par exemple dans la demande de brevet FR-A-2.112.549 et EP-A-504.005 ; les lacases décrites dans les demandes de brevet WO95/07988, WO95/33836, WO95/33837, WO96/00290, WO97/19998 et WO97/19999, dont le contenu fait partie intégrante de la présente description comme par exemple les laccases de Scytalidium, de Polyporus pinsitus, de Myceliophthora thermophila, de Rhizoctonia solani, de Pyricularia orizae, et leurs variantes.

On choira plus préférentiellement les laccases d'origine microbienne ou celles obtenues par biotechnologie.

Dans une forme de réalisation particulièrement préférée de l'invention, l'enzyme utilisée correspond à la tyrosinase (nomenclature EC 1.14.18.1). Par tyrosinase, il faut entendre dans la présente invention toute enzyme présentant une activité tyrosinase, cette enzyme pouvant présenter d'autres activités enzymatiques. L'activité tyrisonase peut se définir comme l'activité enzymatique qui catalyse l'oxydation de la tyrosine pour conduire à la formation du précurseur de mélanine : la Dopaquinone.

A titre d'exemple de sources de tyrosinase, on peut notamment citer la pomme de terre, les champignons, les micro-organismes tels que Neurospora crassa, etc.

La quantité d'enzyme présente dans la composition finale peut varier largement mais est généralement de $5.10^{-3}$ à 5 mg, de préférence $5.10^{-2}$ à 0,5 mg par millilitre de composition finale.

Les précurseurs de colorant pouvant être incorporés dans les compositions de l'invention sont des composés ou mélanges de composés comprenant au moins un cycle aromatique, de préférence un cycle benzénique, comportant au moins deux groupes hydroxyles (OH) portés par deux atomes de carbone consécutifs du cycle aromatique.

Le cycle aromatique peut être un cycle aromatique condensé contenant éventuellement un ou plusieurs hetéroatomes, tel que le naphtalène, le tétrahydronaphtalène, l'indène, l'anthracène, le phénanthrène, l'indole, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine.

Les précurseurs de colorant selon l'invention peuvent être représentés par la formule :

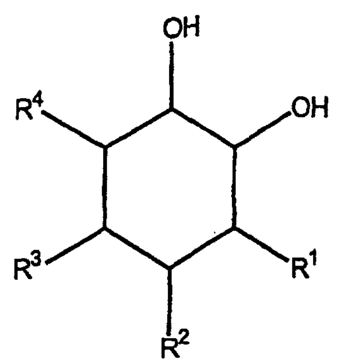

(I)

dans laquelle les substituts $R^1$ à $R^4$, identiques ou différents, représentent un atome d'hydrogène, un radical halogène, hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétéroxyclique éventuellement substitué, un radical contenant un ou plusieurs atomes de silicium, où deux des substituants R1 à R4 forment conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

Les cycles saturés ou insaturés, éventuellement condensés, peuvent être aussi éventuellement substitués.

Les radicaux alkyles sont généralement les radicaux alkyles en $C_1$-$C_{10}$, de préférence les radicaux alkyles en $C_1$-$C_6$, tels que méthyle, éthyle, propyle, butyle, pentyle et hexyle.

Les radicaux alcoxy sont en général les radicaux alcoxy en $C_1$-$C_{20}$, tels que méthoxy, éthoxy, propoxy et butoxy.

Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle, etc.

Les radicaux cycloalkyles sont en général les radicaux cycloalkyles en $C_4$-$C_8$, de préférence les radicaux cyclopentyle et cyclohexyle. Les radicaux cycloalkyles peuvent être des radicaux cycloalkyles substitués, en particulier par des groupes alkyles, alcoxy, acide carboxylique, hydroxyle, amine et cétone.

Les radicaux alcényles sont de préférence des radicaux en $C_1$-$C_{20}$, tels que éthylène, propylène, butylène, pentylène, méthyl-2-propylène et décylène.

Les radicaux contenant un ou plusieurs atomes de silicium sont de préférence des radicaux polydiméthylsiloxane, polydiphenylsiloxane, polydiméthylphénylsiloxane, stéraoxydiméthicone.

Les radicaux hétérocycliques sont en général des radicaux comprenant un ou plusieurs hétéroatomes choisis parmi O, N et S, de préférence O ou N, éventuellement substitués par un ou plusieurs groupes alkyles, alcoxy, acide carboxylique, hydroxyle, amine ou cétone.

Parmi les radicaux hétérocyliques préférés, on peut citer les groupes furyle, pyrannyle, pyrrolyle, imidazolyle, pyrazolyle, pyridyle, thiényle.

De préférence encore, les groupes hétérocycliques sont des groupes condensés tels que des groupes benzofurannyle, chromènyle, xanthényle, indolyle, isoindolyle, quinolyle, isoquinolyle, chromannyle, isochromannyle, indolinyle, isoindolinyle, coumarinyle, isocoumarinyle, ces groupes pouvant être substitués, en particulier par un ou plusieurs groupes OH.

Les précurseurs de colorant préférés sont :

- les flavanols comme la catéchine et le gallate d'épichatéchine,
- les flavonols comme la quercétine,
- les anthocyanidines comme la péonidine,
- les anthocyanines, par exemple l'oenine,
- les hydroxybenzoates, par exemple l'acide gallique,
- les flavones comme la lutéoline,
- les iridoïdes comme l'oleuropéine,

ces produits pouvant être osylés (par exemple glucosylés) et /ou sous forme d'oligomères (procyanidines) ;

- les hydroxystilbènes, par exemple le tétrahydroxy-3,3',4,5'-stilbène, éventuellement osylés (par exemple glucosylés) ;
- la 3,4-dihydroxyphénylalanine et ses dérivés ;
- la 2,3-dihydroxyphenylalanine et ses dérivés ;
- la 4,5-dihydroxyphenylalanine et ses dérivés ;

ces dérivés pouvant être de la forme L (L-DOPA) ou D (D-DOPA),

- le 4,5-dihydroxyindole et ses dérivés ;
- le 5,6-dihydroxyindole et ses dérivés ;
- le 6,7-dihydroxyindole et ses dérivés ;
- le 2,3-dihydroxyindole et ses dérivés ;
- les dihydroxycinnnamates tels que l'acide caféique et l'acide chlorogénique ;
- les hydroxycoumarines ;
- les hydroxyisocoumarines ;
- les hydroxycoumarones;
- les hydroxyisocoumarones;
- les hydroxychalcones ;
- les hydroxychromones ;
- les anthocyanes ;
- les quinones ;

- les hydroxyxanthones ; et
- les mélanges de ceux-ci.

Les polymères formés en particulier avec la catéchine, l'acide gallique et leurs dérivés (tannins) ont des propriétés antimicrobiennes par emprisonnement des microorganismes lors de la polymérisation. Ces tannins ont également des propriétés astringentes intéressantes pour la peau.
En faisant varier la nature des différents précurseurs de colorant et leurs proportions dans la composition, on peut faire varier la couleur de la composition de coloration finale. On obtient ainsi une palette de couleurs.
Par exemple, avec un ratio 1/10 d'acide chlorogénique et de catéchine, on obtient une coloration marron claire et avec un ratio 1/1 une coloration acajou.
Les précurseurs de colorants peuvent être des extraits de plantes, fruits, agrumes, légumes et des mélanges de ces extraits, qui contiennent de nombreux polyphénols tels que définis précédemment.
Parmi les extraits de plantes, on peut citer les extraits de rose et de thé.
Parmi les extraits de fruits, on peut citer les extraits de pomme, de raisin (en particulier de pépins de raisin) et de banane.
Parmi les extraits de légumes, on peut citer l'extrait de pomme de terre.
Suivant les parties de fruits utilisés, par exemple pulpe ou pépins de raisin, la coloration obtenue est différente.
On peut également utiliser des mélanges d'extraits de plantes et/ou de fruits tels que des mélanges d'extraits de pomme et de thé et des mélanges d'extraits de raisin et de pomme.
La quantité de précurseur de colorant dans la composition finale doit être suffisante pour obtenir une coloration visible. Cette quantité peut varier dans de larges mesures en fonction de la nature du précurseur et de l'intensité voulue pour la coloration.
En général, on obtiendra une coloration convenable lorsque la quantité de précurseur de colorant est telle que la teneur en précurseur de colorant dans la composition de coloration finale est d'au moins 10 micromoles par millilitre de composition finale.
Bien évidemment, les doses des constituants des compositions de l'invention sont non toxiques et compatibles avec le cosmétique.
Les compositions selon l'invention peuvent également contenir une quantité efficace d'au moins un acide aminé comportant au moins un groupe thiol (SH) et de préférence un seul groupe thiol, ces acides aminés pouvant être sous la forme de chlorhydrates.
Les acides aminés préférés selon l'invention sont les acides aminés qui contiennent une fonction amine en position $\alpha$ par rapport à une foncction acide carboxylique.
Les acides aminés préférés peuvent être représentés par la formule :

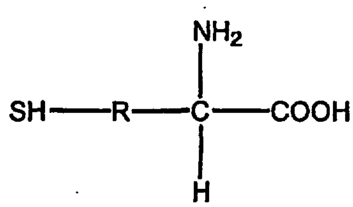

(II)

dans laquelle R est un radical hydrocarboné divalent, linéaire ou ramifié, par exemple en $C_1$-$C_{10}$, de préférence en $C_1$-$C_6$, tel qu'un radical méthylène, éthylène, butylène, éthylidène, propylidène, un radical cyclique saturé divalent, éventuellement substitué, par exemple en $C_4$-$C_8$, un groupe aromatique divalent, éventuellement substitué, tel qu'un radical phénylène, tolylène, xylylène.
Parmi les acides aminés préférés pour les compositions de l'invention, on peut citer la cystéine et ses dérivés, en particulier la L-cystéine et le chlorhydrate de L-cystéine, et le glutathion et ses dérivés.
Selon une composition préférée de l'invention, le précurseur de colorant est la L-DOPA ou un dihydroxyindole, et l'acide aminé, la L-cystéine ou le glutation.
Les proportions relatives d'acide aminé et de précurseur de colorant d'oxydation dans les compositions de l'invention peuvent varier dans de larges mesures en fonction de la coloration voulue. Généralement, le rapport molaire acide aminé/précurseur de colorant variera de 0,001 à 50, de préférence de.0,01 à 5 et mieux de 0,05 à 2,5.
En général, la teneur en acide aminé à groupe thiol dans la composition finale lorsqu'il est présent, est d'au moins 0,01 micromole par millilitre, de préférence au moins 0,1 micromole/ml.
En faisant varier la nature et les proportions des précurseurs de colorant et des acides aminés éventuels de la composition, on peut obtenir toute une palette de teintes et en particulier des teintes proches de celles du bronzage

naturel.

Ainsi, l'association de la cystéine et de dihydroxyindole permet d'obtenir une nuance marron, plus proche de celle d'un bronzage naturel que l'emploi du seul dihydroxyindole qui conduit à une couleur uniquement noire.

Cette association permet d'avoir une coloration naturelle de la peau et du cheveu qui et un mélange d'eulamine et de phéomélanine en proportions variables.

Les compositions de l'invention peuvent également conduire à des films colorés qui présentent une certaine transparence. Cette caractéristique, alliée au fait que ces compositions permettent d'obtenir une teinte proche du bronzage naturel, et qu'elles activent la pigmentation naturelle de la peau, rend ces compositions particulièrement intéressantes pour l'application au bronzage de la peau, permettant de combiner bronzage artificiel et bronzage naturel tout en obtenant une teinte homogène.

Le milieu physiologiquement acceptable est un milieu solide ou liquide ne nuisant pas à la propriété de coloration des précurseurs ni à l'effet du système catalytique.

Le milieu physiologiquement acceptable est de préférence un milieu solubilisant du précurseur de colorant de l'enzyme et de l'acide aminé éventuel.

De préférence encore, le milieu physiologiquement acceptable est un milieu bactériostatique, ce milieu acceptable comprenant un solvant ou un mélange de solvants.

Parmi les solvants des précurseurs convenant pour la formulation des compositions selon l'invention, on peut citer l'eau déminéralisée, de préférence ayant une conductivité à température ambiante (25°C) inférieure ou égale à 25 mΩn, par exemple l'eau fabriquée et/ou distribuée par la société MILLIPORE sous la dénomination milli Q, les alcools, les solvants polaires et leurs mélanges.

Les alcools sont de préférence des alcanols inférieurs ($C_1$-$C_6$) tels que l'éthanol et l'isopropanol et les alcanediols tels que l'éthylèneglycol, le propylène glycol et le pentane diol.

Parmi les solvants polaires, on peut citer les éthers, les esters (en particulier les acétates), le diméthylsulfoxyde (DMSO), la N-méthylpyrrolidone (NMP), les cétones (en particulier l'acétone) et leurs mélanges.

Le milieu physiologiquement acceptable comprend de préférence de l'eau déminéralisée ou un mélange eau/alcool, en particulier eau/éthanol.

La quantité d'alcool dans le mélange eau/alcool peut représenter jusqu'à 80% en poids du mélange eau/alcool, de préférence 1 à 50% en poids et mieux 5 à 20% en poids.

Le milieu physiologiquement acceptable peut être un milieu solide tel qu'un excipient pour la formulation de galets et comprimés, en particulier effervescents.

Les compositions selon l'invention peuvent également comprendre tout adjuvant classique, en proportion usuelle, qui ne nuit pas aux propriétés recherchées.

Les compositions de l'invention peuvent par exemple comporter des filtres solaires et de protection contre les UV, tels que les filtres organiques comme le benzophénone, le benzylidène, les dérivés de triazine, les dérivés de l'hydroxyphényl benzotriazole, les dérivés de l'acide cinnamique, les dérivés d'oxybenzone, l'octocrylène, les dérivés de benzilidène-camphre et les filtres minéraux tels que ZnO, $TiO_2$.

Toutefois, dans les compositions de l'invention utilisée dans des formulations pour le bronzage, les pigments formés (mélanines) par l'association du système catalytique et de l'enzyme peuvent assurer une protection solaire en l'absence de filtres additionnels.

De préférence, les compositions selon l'invention sont exemptes d'agents de chélation des sels de Mn(II) et/ou Zn(II) utilisés, car ces agents tendent à inhiber l'oxydation des précurseurs de colorant.

Pour révéler la coloration des compositions suivant l'invention, il suffit de mettre la composition selon l'invention en présence d'un milieu oxydant tel qu'un milieu contenant de l'oxygène (par exemple l'oxygène de l'air).

Les compositions selon l'invention sont utiles pour la coloration de la peau et/ou des matières kératiniques tels que les cheveux, les cils, les sourcils et les poils.

Pour la coloration de la peau et des fibres kératiniques, différents procédés d'application des compositions selon l'invention peuvent être utilisés.

Selon un premier procédé, on applique sur la peau ou les fibres kératiniques, en présence d'oxygène, par exemple l'oxygène de l'air, une composition comprenant tous les ingrédients de la composition, c'est-à-dire l'enzyme et le système catalytique et éventuellement le ou les précurseurs de colorant et/ou le ou les acides aminés à groupe thiol. Lorsque la composition contient un précurseur de colorant, la révélation de la couleur peut se faire soit avant l'application, soit après application de la composition sur la peau et/ou les fibres kératiniques.

Selon un second procédé, on peut en premier lieu appliquer sur la peau ou les matières kératiniques un film d'une composition de base, d'une enzyme et éventuellement d'un ou plusieurs précurseurs de colorant et d'un ou plusieurs acides aminés à groupe thiol dans un milieu physiologiquement acceptable, puis sur le film de composition de base, un film du système catalytique dans un milieu physiologiquement acceptable, qui en présence d'oxygène, révélera la coloration de la composition de base.

On peut bien évidemment inverser l'ordre d'application des films.

L'application des films peut se faire par tout moyen connu, en particulier par pulvérisation.

Une fois la composition selon l'invention appliquée sur la peau et/ou les fibres kératiniques, on peut appliquer une solution à un ou plusieurs orthodiphénols, et dans la mesure où le système catalytique initialement présent n'a pas été totalement consommé, l'apport de cet (ou ces) orthodiphénol(s) supplémentaire(s) augmentera la rémanence de la coloration et/ou selon le (ou les) orthodiphénol(s) utilisé(s) nuancera la teinte et/ou en augmentera l'intensité.

Les compositions de l'invention peuvent également permettre de masquer des défauts de pigmentation tels que le vitiligo, le masque de grossesse, ainsi que des imperfections de la peau comme les taches de vieillissement et la couperose.

La coloration de la composition peut être déterminée par le choix du précurseur de colorant et/ou de l'acide aminé à groupe thiol en fonction du défaut à masquer.

Les compositions de l'invention présentent l'avantage de ne pas nécessiter l'emploi de peroxyde d'hydrogène.

Les compositions selon l'invention peuvent se présenter sous des formes diverses, telles que sous forme de liquides, de crèmes, de gels, ou sous toute autre forme appropriée pour réaliser une coloration, notamment de la peau et des matières kératiniques.

Selon une première réalisation, les compositions selon l'invention peuvent être conditionnées sous forme d'aérosol à un seul compartiment dans lequel se trouvent la composition renfermant la ou les enzymes, éventuellement le ou les précurseurs de colorant et/ou le ou les acides aminés à groupe SH, le système catalytique, et un gaz propulseur inerte classique tel que de l'azote, un hydrocarbure saturé comme l'isopropane ou un hydrocarbure fluoré, par exemple un Fréon®.

Dans une seconde réalisation, la composition selon l'invention peut être conditionnée sous forme d'un kit comportant deux conteneurs distincts, l'un pour la composition de base contenant la ou les enzymes, éventuellement le ou les précurseurs de colorant et/ou le ou les acides aminés, l'autre pour le système catalytique, la composition de base et le système catalytique étant mélangés ou appliqués successivement au moment de l'emploi.

Dans une troisième réalisation, la composition peut être contenue dans un système à pompe à un seul compartiment, sans reprise d'air; ou dans un système à pompe à deux compartiments, la composition de base étant dans un compartiment et le système catalytique dans l'autre.

Dans une quatrième réalisation, la composition selon l'invention peut se présenter sous forme de galets, en particulier pour le bain. Chaque galet peut comporter, mélangé à un excipient, la ou les enzymes, éventuellement le ou les précurseurs de coloration et/ou le ou les acides aminés et le système catalytique, l'excipient empêchant la réaction en présence d'oxygène ou encore la ou les enzymes, éventuellement le ou les précurseurs de colorant et/ou le ou les acides aminés d'une part et le système catalytique d'autre part sont contenus dans des galets distincts.

En délitant soit le galet unique soit un galet de chacun des constituants dans de l'eau, par exemple l'eau d'un bain, on réalise la composition colorante selon l'invention.

Les galets, comme cela est classique, peuvent être des galets effervescents.

L'excipient utilisé peut être tout excipient classique tel qu'un mélange de talc, de stéarate (en particulier de magnésium), d'acide citrique et/ou tartrique, et d'hydrogénocarbonate alcalin et/ou alcalino terreux.

La quantité d'acide citrique et/ou tartrique présente doit être telle qu'il n'y ait pas une neutralisation de l'hydrogénocarbonate résultant en un manque d'hydrogénocarbonate libre par rapport au Mn(II) et/ou Zn(II).

D'autre part, comme l'eau, en particulier l'eau du robinet et certaines eaux de source ou minérales, contiennent généralement du manganèse (II), il est parfois suffisant de mettre dans l'eau un seul galet contenant de l'hydrogénocarbonate, la ou les enzymes et éventuellement le ou les précurseurs de coloration et/ou le ou les acides aminés à groupe thiol, la teneur en Mn(II) du système catalytique étant alors fournie par le Mn(II) présent dans l'eau.

De même, certains extraits végétaux (par exemple des extraits de feuilles de thé) peuvent contenir des quantités importantes de manganèse (II). En fonction de ces teneurs, un ajustement des concentrations du système catalytique est effectué de manière à avoir un résultat satisfaisant.

Bien évidemment, on peut faire varier l'intensité de la coloration en délitant plusieurs galets dans l'eau.

D'autre part, la vitesse de coloration peut être accélérée en ajoutant à la composition un composé ou une formulation de composés engendrant de l'oxygène, par exemple par contact avec de l'eau. Ainsi, on peut incorporer un tel composé ou formulation, par exemple du peroxyde de sodium, dans un galet. Les exemples suivants illustrent la présente invention. Dans les exemples, sauf indication contraire, tous les pourcentages et parties sont exprimés en poids.

EXEMPLE 1

Cet exemple démontre « in vitro » l'augmentation de la vitesse de formation des composés colorés (mélanine).

Dans une plaque à 96 puits, on a placé des échantillons de 200 µl (4 puits par concentration de produit) dont les formulations sont données dans le Tableau I ci-dessous.

La plaque est incubée 1 heure à 30°C puis on mesure la densité optique (DO) des échantillons à 620 nm

avec une lenteur de densité optique pour microplaque (Labsystem iEMS). Les résultats sont donnés dans le Tableau II.

## TABLEAU I

| | C | A1 | A2 | A3 | B1 | B2 | B3 | 1 | 2 | 3 |
|---|---|---|---|---|---|---|---|---|---|---|
| Solution de L-tyrosine à 500µM/l dans tampon phosphate de Dulbecco's | 100µl | 100µl | → | | | | | | | |
| Solution de tyrosinase à 0,1mg/ml dans tampon phosphate de Dulbecco's (Tyrosinase SIGMAT 7755 à 3300 unités par mg) | 10µl | 10µl | → | | | | | | | |
| $NaHCO_3$ | - | 50mM/l | 100mM/l | 250mM/l | - | - | - | 50mM/l | 100mM/l | 250mM/l |
| $MnCl_2$ | - | - | - | - | 500µM/l | 1000µM/l | 2500µM/l | 500µM/l | 1000µM/l | 2500µM/l |
| Tampon phosphate de Dulbecco's q.s.p. | 200µl | 200µl | → | | | | | | | |

Seuls les exemples 1,2 et 3 illustrent l'invention.

TABLEAU II

| Exemple N° | Densité optique | % augmentation par rapport au contrôle C |
|---|---|---|
| C | 0,1708 | - |
| A1 | 0,257 | 51 |
| A2 | 0,275 | 61 |
| A3 | 0,257 | 51 |
| B1 | 0,168 | NS |
| B2 | 0,166 | NS |
| B3 | 0,173 | NS |
| 1 | 0,271 | 59 |
| 2 | 0,295 | 73 |
| 3 | 0,291 | 71 |
| N.S. : non significatif | | |

Les exemples montrent que l'utilisation d'un système catalytique selon l'invention accroît grandement l'activité progimentation de l'enzyme.

## Revendications

1. Composition pour la coloration de la peau et/ou des fibres kératiniques, **caractérisée en ce qu'**elle comprend, dans un milieu physiologiquement acceptable, une quantité efficace d'au moins une enzyme ayant une activité propigmentation et une quantité efficace d'un système catalytique comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges, les proportions du premier et du second constituant étant telles que :

$$\frac{[Mn(II)]}{[HCO_3]} \leq 1 \text{ avec } [Mn(II)] \neq 0$$

$$\frac{[Zn(II)]}{[HCO_3]} \leq 1 \text{ avec} [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et [$HCO_3$] représentent respectivement les concentrations molaires en Mn(II), Zn(II) et $HCO_3$ dans la composition, et facultativement :

- une quantité efficace d'au moins un précurseur de colorant choisi parmi les composés comportant au moins un cycle aromatique ayant au moins deux groupes hydroxyles portés par deux atomes de carbone consécutifs du cycle aromatique et/ou,
- une quantité efficace d'au moins un acide aminé comportant au moins un groupe thiol.

2. Composition selon la revendication 1, **caractérisée en ce que** le rapport $\frac{[Mn(II)]}{[HCO_3]}$ varie de $10^{-5}$ à $10^{-1}$, de préférence de $10^{-3}$ à $10^{-2}$ et mieux est de l'ordre de $5.10^{-3}$.

3. Composition selon la revendication 1 ou 2, **caractérisée en ce que** le rapport $\frac{[Zn(II)]}{[HCO_3]}$ varie de $10^{-4}$ à < 1, de

préférence de $10^{-3}$ à < 1, et mieux est de l'ordre de $5.10^{-1}$.

**4.** Composition selon l'une quelconque des revendications précédentes, **caractérisée en ce que** le rapport $\frac{[Mn(II) + Zn(II)]}{[HCO_3]}$ varie de $10^{-5}$ à $10^{-1}$, de préférence $10^{-3}$ à $10^{-2}$.

**5.** Composition selon l'une quelconque des revendications précédentes, **caractérisée en ce que** les sels de Mn(II) et de Zn(II) sont choisis parmi les chlorure, fluorure, iodure, sulfate, phosphate, nitrate, perchlorate, les sels d'acides carboxyliques et leurs mélanges.

**6.** Composition selon la revendication 5, **caractérisée en ce que** le sel de Mn(II) et/ou de Zn(II) est le chlorure.

**7.** Composition selon la revendication 5, **caractérisée en ce que** les sels d'acides carboxyliques sont des sels d'acides carboxyliques hydroxylés.

**8.** Composition selon la revendication 7, **caractérisée en ce que** le sel d'acide carboxylique hydroxylé est le gluconate.

**9.** Composition selon l'une quelconque des revendications précédentes, **caractérisée en ce que** l'hydrogénocarbonate est choisi parmi l'hydrogénocarbonate de sodium, l'hydrogénocarbonate de potassium, l'hydrogénocarbonate de magnésium, l'hydrogénocarbonate de calcium et leurs mélanges.

**10.** Composition selon l'une quelconque des revendications précédentes, **caractérisée en ce que** l'enzyme est choisie parmi les pyranose oxydases, les glucose oxydases, les glycérol oxydases, les lactate oxydases, les pyruvate oxydases, les uricases, les choline oxydases, les sarcosine oxydases, les bilirubines oxydases, les laccases, les tyrosinases, les péroxydases, les catalases, les superoxydesdimutases et leurs mélanges, ou parmi des extraits végétaux ou animaux contenant des enzymes précitées.

**11.** Composition selon la revendication 10, **caractérisée en ce que** l'enzyme est choisie parmi les tyrosinases.

**12.** Composition selon l'une quelconque des revendications précédentes, **caractérisée en ce qu'**elle comprend $5.10^{-3}$ à 5mg, de préférence $5.10^{-2}$ à 0.5mg d'enzyme par millilitre de composition finale.

**13.** Composition selon l'une quelconque des revendications précédentes, **caractérisée en ce que** le cycle aromatique comportant au moins deux groupes hydroxyle sur deux atomes de carbone consécutifs du précurseur de colorant est un cycle benzénique ou un cycle aromatique condensé.

**14.** Composition selon la revendication 13, **caractérisée en ce que** le précurseur de colorant est un composé ou un mélange de composés de formule :

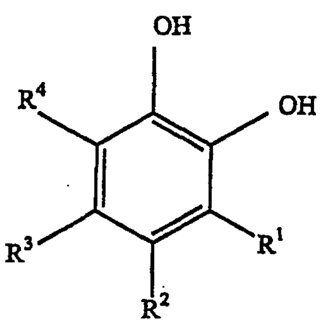

(I)

dans laquelle les substituts $R^1$ à $R^4$, identiques ou différents, représentent un atome d'hydrogène, un halogène, un radical hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvait être éventuellement substitué, aryle, aryle substitué, un radical hétérocyclique éventuellement substitué, un radical contenant éventuellement un ou plusieurs atomes de silicium, où deux des substituants $R^1$ à $R^4$ peuvent former conjointement un cycle saturé ou insaturé contenant éventuellement un ou plusieurs hétéroatomes et éventuellement condensé avec un ou plusieurs cycles saturés

ou insaturés contenant éventuellement un ou plusieurs hétéroatomes.

**15.** Composition selon l'une quelconque des revendications précédentes, **caractérisée en ce que** le précurseur de colorant est choisi parmi les flavanols, les flavonols, les anthocyaninidines, les anthocyanines, les hydroxybenzoates, les flavones, les iridoïdes, ces composés pouvant être éventuellement osylés et/ou sous forme d'oligomères, les hydroxystilbènes éventuellement osylés, la 3,4-dihydroxyphénylalanine et ses dérivés, la 2,3-dihydroxyphénylalanine et ses dérivés, la 4,5-dihydroxyphénylalanine et ses dérivés, le 4,5-dihydroxyindole et ses dérivés, le 5,6-dihydroxyindole et ses dérivés, le 6,7-dihydroxyindole et ses dérivés, le 2,3-dihydroxyindole et ses dérivés, les dihydroxycinnamates, les hydroxycoumarines, les hydroxyisocoumarines, les hydroxycoumarones, les hydroxyisocoumarones, les hydroxychalcones, les hydroxychromones, les anthocyanes, les quinones, les hydroxyxantones, et les mélanges de deux ou plus des composés précédents.

**16.** Composition selon l'une quelconque des revendications précédentes, **caractérisée en ce que** le précurseur de colorant est choisi parmi les extraits de plantes, de fruits, d'agrumes, de légumes et leurs mélanges.

**17.** Composition selon la revendication 16, **caractérisée en ce que** le précurseur de colorant est choisi parmi les extraits de thé, de raisin, de pomme, de banane, de pomme de terre et leurs mélanges.

**18.** Composition selon l'une quelconque des revendications précédentes, **caractérisée en ce que** le précurseur de colorant est présent en une quantité d'au moins 10 micromoles par millilitre de composition.

**19.** Composition selon l'une quelconque des revendications précédentes, **caractérisée en ce que** le ou les acides aminés comprenant au moins un groupe thiol sont choisis parmi les acides aminés ayant une fonction amine en position $\alpha$ par rapport à une fonction acide carboxylique.

**20.** Composition selon la revendication 19, **caractérisée en ce que** le ou les acides aminés sont choisis parmi la cystéine et ses dérivés et le glutathion et ses dérivés.

**21.** Composition selon l'une des revendications 19 à 20, **caractérisée en ce** le rapport molaire du (ou des) acide(s) aminé(s) à groupe SH au(x) précurseur(s) de colorant(s) varie de 0,001 à 50, de préférence de 0,01 à 5, et mieux de 0,05 à 2,5.

**22.** Composition selon l'une quelconque des revendications 19 à 21, **caractérisée en ce que** le précurseur de colorant est la L-DOPA ou un dihydroxyindole et l'acide aminé, la L-cystéine ou le glutathion.

**23.** Composition selon l'une quelconque des revendications précédentes, **caractérisée en ce que** le milieu physiologiquement acceptable est un milieu solubilisant de l'enzyme et du précurseur de colorant et de l'acide aminé éventuels, de préférence à propriété bactériostatique.

**24.** Composition selon la revendication 23, **caractérisée en ce que** le milieu physiologiquement acceptable comprend un solvant ou un mélange de solvants.

**25.** Composition selon la revendication 24, **caractérisée en ce que** le solvant est choisi parmi l'eau déminéralisée, les alcools, les éthers, le diméthylsulfoxyde, la N-méthylpyrrolidone, les cétones et leurs mélanges.

**26.** Composition selon la revendication 25, **caractérisée en ce que** l'alcool est un alcanol ou un alcanediol.

**27.** Composition selon la revendication 25, **caractérisée en ce que** le solvant est un mélange eau déminéralisée/alcool.

**28.** Composition selon la revendication 27, **caractérisée en ce que** l'alcool représente jusqu'à 80% en poids du mélange, de préférence 1 à 50% en poids et mieux de 5 à 20% en poids.

**29.** Composition selon l'une quelconque des revendications précédentes, **caractérisée en ce qu'**elle est exempte de tout agent de chélation du sel de Mn(II) et/ou Zn(II).

**30.** Composition selon l'une quelconque des revendications précédentes, **caractérisée en ce qu'**elle se présente sous la forme de deux composants séparés, un premier composant comprenant le système catalytique dissous

dans un milieu physiologiquement acceptable et un deuxième composant comprenant l'enzyme et éventuellement le précurseur de colorant et l'acide aminé à groupe thiol, dissous dans un milieu physiologiquement acceptable.

**31.** Composition selon l'une quelconque des revendications 1 à 29, **caractérisée en ce qu'**elle est conditionnée sous forme d'un aérosol ou d'un système à pompe sans reprise d'air.

**32.** Composition selon la revendication 30, **caractérisée en ce qu'**elle est conditionnée dans un système à pompe à deux compartiments distincts, chacun des composants étant contenu séparément dans un des deux compartiments.

**33.** Composition selon l'une quelconque des revendications 1 à 22, **caractérisée en ce qu'**elle est conditionnée sous forme d'un galet.

**34.** Composition selon la revendication 33, **caractérisée en ce que** le comprimé comprend un excipient contenant de l'acide citrique et /ou tartrique en quantité sous stoechiométrique par rapport à l'hydrogénocarbonate alcalin et/ou alcalino terreux.

**35.** Composition selon l'une quelconque des revendication 1 à 22, **caractérisée en ce qu'**elle est conditionnée sous forme de deux galets, un premier galet comprenant le système catalytique et un excipient et un second galet comprenant l'enzyme et éventuellement le précurseur de colorant et l'acide aminé à groupe thiol éventuel, dans un excipient.

**36.** Composition selon l'une quelconque des revendications 33 à 35, **caractérisée en ce que** le ou les galets sont des galets effervescents.

**37.** Procédé de coloration de la peau et/ou des fibres kératiniques, **caractérisé en ce qu'**il consiste à appliquer sur la peau et/ou les fibres kératiniques une couche d'une composition selon l'une quelconque des revendications 1 à 32.

**38.** Procédé selon la revendication 37, **caractérisé en ce que** la couche est obtenue en appliquant sur la peau et/ou les fibres kératiniques un premier film d'une composition de base contenant la (ou les) enzyme(s), le (ou les) éventuel(s) précurseur(s) de colorant, et le (ou les) éventuel(s) acide(s) aminé(s) à groupe thiol, dans un milieu physiologiquement acceptable et en appliquant sur le premier film un second film d'une composition catalytique comprenant le système catalytique dans un milieu physiologiquement acceptable et vice-versa.

**39.** Procédé selon la revendication 37 ou 38, **caractérisé en ce que** les films sont appliqués par pulvérisation.

**Patentansprüche**

**1.** Zusammensetzung zum Färben der Haut und/oder von Keratinfasern, **dadurch gekennzeichnet, dass** sie in einem physiologisch annehmbaren Medium folgendes umfasst: eine wirksame Menge wenigstens eines Enzyms mit einer Propigmentierungswirkung und eine wirksame Menge eines Katalysatorsystems mit einem ersten Bestandteil, der aus den Salzen und Oxiden von Mn(II) und/oder Zn(II) und ihren Gemischen ausgewählt ist, und einem zweiten Bestandteil, der aus Alkalimetallhydrogencarbonaten, Erdalkalimetallhydrogencarbonaten und ihren Gemischen ausgewählt ist, wobei die Anteile des ersten und des zweiten Bestandteils so groß sind, dass:

$$\frac{[Mn(II)]}{[HCO_3]} \leq 1 \text{ mit } [Mn(II)] \neq 0$$

$$\frac{[Zn(II)]}{[HCO_3]} \leq 1 \text{ mit } [Zn(II)] \neq 0$$

$$\frac{[Mn(II) + Zn(II)]}{[HCO_3]} \leq 1 \text{ mit } [Mn(II)] \text{ und } [Zn(II)] \neq 0$$

wobei [Mn(II)], [Zn(II)] und [$HCO_3$] die molaren Konzentrationen von Mn(II), Zn(II) bzw. $HCO_3$ in der Zusammensetzung darstellen; sowie fakultativ:

- eine wirksame Menge wenigstens eines Farbstoffvorläufers, der aus den Verbindungen ausgewählt ist, die wenigstens einen aromatischen Ring umfassen, der wenigstens zwei Hydroxygruppen aufweist, die an zwei benachbarte Kohlenstoffatome des aromatischen Rings gebunden sind; und/oder

- eine wirksame Menge wenigstens einer Aminosäure, die wenigstens eine Thiolgruppe umfasst.

2. Zusammensetzung gemäß Anspruch 1, **dadurch gekennzeichnet, dass** das Verhältnis [Mn(II)]/[$HCO_3$] von $10^{-5}$ bis $10^{-1}$, vorzugsweise $10^{-3}$ bis $10^{-2}$, variiert und besonders bevorzugt in der Größenordnung von $5 \cdot 10^{-3}$ liegt.

3. Zusammensetzung gemäß Anspruch 1 oder 2, **dadurch gekennzeichnet, dass** das Verhältnis [Zn(II)]/[$HCO_3$] von $10^{-4}$ bis < 1, vorzugsweise $10^{-3}$ bis < 1, variiert und besonders bevorzugt in der Größenordnung von $5 \cdot 10^{-1}$ liegt.

4. Zusammensetzung gemäß einem der vorstehenden Ansprüche, **dadurch gekennzeichnet, dass** das Verhältnis [Mn(II) + Zn(II)]/[$HCO_3$] von $10^{-5}$ bis $10^{-1}$, vorzugsweise $10^{-3}$ bis $10^{-2}$, variiert.

5. Zusammensetzung gemäß einem der vorstehenden Ansprüche, **dadurch gekennzeichnet, dass** die Salze von Mn(II) und Zn(II) aus dem Chlorid, Fluorid, Iodid, Sulfat, Phosphat, Nitrat, Perchlorat, den Salzen von Carbonsäuren und ihren Gemischen ausgewählt sind.

6. Zusammensetzung gemäß Anspruch 5, **dadurch gekennzeichnet, dass** es sich bei dem Salz von Mn(II) und/oder Zn(II) um das Chlorid handelt.

7. Zusammensetzung gemäß Anspruch 5, **dadurch gekennzeichnet, dass** die Salze von Carbonsäuren Salze von Hydroxycarbonsäuren sind.

8. Zusammensetzung gemäß Anspruch 7, **dadurch gekennzeichnet, dass** es sich bei dem Salz der Hydroxycarbonsäure um Gluconat handelt.

9. Zusammensetzung gemäß einem der vorstehenden Ansprüche, **dadurch gekennzeichnet, dass** das Hydrogencarbonat aus Natriumhydrogencarbonat, Kaliumhydrogencarbonat, Magnesiumhydrogencarbonat, Calciumhydrogencarbonat und ihren Gemischen ausgewählt ist.

10. Zusammensetzung gemäß einem der vorstehenden Ansprüche, **dadurch gekennzeichnet, dass** das Enzym ausgewählt ist aus Pyranose-Oxidasen, Glucose-Oxidasen, Glycerin-Oxidasen, Lactat-Oxidasen, Pyruvat-Oxidasen, Uricasen, Cholin-Oxidasen, Sarcosin-Oxidasen, Bilirubin-Oxidasen, Laccasen, Tyrosinasen, Peroxidasen, Katalasen, Superoxid-Dismutasen und ihren Gemischen oder aus Pflanzen- oder Tierextrakten, die die genannten Enzyme enthalten.

11. Zusammensetzung gemäß Anspruch 10, **dadurch gekennzeichnet, dass** das Enzym aus Tyrosinasen ausgewählt ist.

12. Zusammensetzung gemäß einem der vorstehenden Ansprüche, **dadurch gekennzeichnet, dass** sie $5 \cdot 10^{-3}$ bis 5 mg, vorzugsweise $5 \cdot 10^{-2}$ bis 0,5 mg, Enzym pro Milliliter der endgültigen Zusammensetzung umfasst.

13. Zusammensetzung gemäß einem der vorstehenden Ansprüche, **dadurch gekennzeichnet, dass** der aromatische Ring, der wenigstens zwei Hydroxygruppen an zwei benachbarten Kohlenstoffatomen aufweist, des Farbstoffvorläufers ein benzolischer Ring oder ein kondensierter aromatischer Ring ist.

14. Zusammensetzung gemäß Anspruch 13, **dadurch gekennzeichnet, dass** der Farbstoffvorläufer eine Verbindung oder ein Gemisch von Verbindungen der folgenden Formel ist:

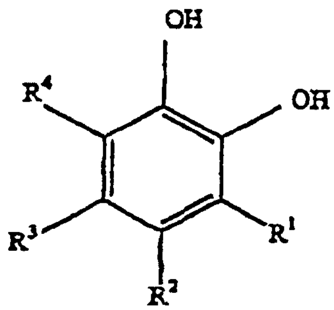

(I)

wobei die Substituenten $R^1$ bis $R^4$ gleich oder verschieden sind und folgendes darstellen: ein Wasserstoffatom, ein Halogen, eine Hydroxy-, Carboxy-, Alkylcarboxylatgruppe, gegebenenfalls substituiertes Amino, gegebenenfalls substituiertes lineares oder verzweigtes Alkyl, gegebenenfalls substituiertes lineares oder verzweigtes Alkenyl, gegebenenfalls substituiertes Cycloalkyl, Alkoxy, Alkoxyalkyl, Alkoxyaryl, wobei die Arylgruppe gegebenenfalls substituiert sein kann, Aryl, substituiertes Aryl, ein gegebenenfalls substituierter heterocyclischer Rest, ein Rest, der gegebenenfalls ein oder mehrere Siliciumatome enthält, wobei zwei der Substituenten $R^1$ bis $R^4$ zusammen einen gesättigten oder ungesättigten Ring bilden können, der gegebenenfalls ein oder mehrere Heteroatome enthält und gegebenenfalls mit einem oder mehreren gesättigten oder ungesättigten Ringen, die gegebenenfalls ein oder mehrere Heteroatome enthalten, kondensiert ist.

**15.** Zusammensetzung gemäß einem der vorstehenden Ansprüche, **dadurch gekennzeichnet, dass** der Farbstoffvorläufer ausgewählt ist aus Flavanolen, Flavonolen, Anthocyaninidinen, Anthocyaninen, Hydroxybenzoaten, Flavonen, Iridoiden, wobei diese Verbindungen gegebenenfalls osyliert sein und/oder in Form von Oligomeren vorliegen können, gegebenenfalls osylierten Hydroxystilbenen, 3,4-Dihydroxyphenylalanin und seinen Derivaten, 2,3-Dihydroxyphenylalanin und seinen Derivaten, 4,5-Dihydroxyphenylalanin und seinen Derivaten, 4,5-Dihydroxyindol und seinen Derivaten, 5,6-Dihydroxyindol und seinen Derivaten, 6,7-Dihydroxyindol und seinen Derivaten, 2,3-Dihydroxyindol und seinen Derivaten, Dihydroxycinnamaten, Hydroxycumarinen, Hydroxyisocumarinen, Hydroxycumaronen, Hydroxyisocumaronen, Hydroxychalconen, Hydroxychromonen, Anthocyanen, Chinonen, Hydroxyxanthonen und den Gemischen von zwei oder mehreren der genannten Verbindungen.

**16.** Zusammensetzung gemäß einem der vorstehenden Ansprüche, **dadurch gekennzeichnet, dass** der Farbstoffvorläufer aus Extrakten von Pflanzen, Früchten, Zitrusfrüchten, Gemüsen/Feldfrüchten und ihren Gemischen ausgewählt ist.

**17.** Zusammensetzung gemäß Anspruch 16, **dadurch gekennzeichnet, dass** der Farbstoffvorläufer aus Extrakten von Tee, Weintraube, Apfel, Banane, Kartoffel und ihren Gemischen ausgewählt ist.

**18.** Zusammensetzung gemäß einem der vorstehenden Ansprüche, **dadurch gekennzeichnet, dass** der Farbstoffvorläufer in einer Menge von wenigstens 10 $\mu$mol pro Milliliter der Zusammensetzung vorhanden ist.

**19.** Zusammensetzung gemäß einem der vorstehenden Ansprüche, **dadurch gekennzeichnet, dass** die Aminosäure oder -säuren, die wenigstens eine Thiolgruppe umfassen, aus Aminosäuren, die eine Aminofunktion in $\alpha$-Position in Bezug auf eine Carbonsäurefunktion aufweisen, ausgewählt sind.

**20.** Zusammensetzung gemäß Anspruch 19, **dadurch gekennzeichnet, dass** die Aminosäure oder -säuren aus Cystein und seinen Derivaten sowie Glutathion und seinen Derivaten ausgewählt sind.

**21.** Zusammensetzung gemäß einem der Ansprüche 19 bis 20, **dadurch gekennzeichnet, dass** das Stoffmengenverhältnis der Aminosäure oder

- säuren mit der SH-Gruppe zu dem bzw. den Farbstoffvorläufern von 0,001 bis 50, vorzugsweise 0,01 bis 5 und besonders bevorzugt 0,05 bis 2,5 variiert.

**22.** Zusammensetzung gemäß einem der Ansprüche 19 bis 21, **dadurch gekennzeichnet, dass** es sich bei dem Farbstoffvorläufer um L-DOPA oder ein Dihydroxyindol und bei der Aminosäure um L-Cystein oder Glutathion

handelt.

23. Zusammensetzung gemäß einem der vorstehenden Ansprüche, **dadurch gekennzeichnet, dass** das physiologisch annehmbare Medium ein Medium ist, das das Enzym und gegebenenfalls den Farbstoffvorläufer und die Aminosäure auflöst, vorzugsweise mit bakteriostatischer Eigenschaft.

24. Zusammensetzung gemäß Anspruch 23, **dadurch gekennzeichnet, dass** das physiologisch annehmbare Medium ein Lösungsmittel oder ein Gemisch von Lösungsmitteln umfasst.

25. Zusammensetzung gemäß Anspruch 24, **dadurch gekennzeichnet, dass** das Lösungsmittel aus entmineralisiertem Wasser, Alkoholen, Ethern, Dimethylsulfoxid, N-Methylpyrrolidon, Ketonen und ihren Gemischen ausgewählt ist.

26. Zusammensetzung gemäß Anspruch 25, **dadurch gekennzeichnet, dass** der Alkohol ein Alkanol oder ein Alkandiol ist.

27. Zusammensetzung gemäß Anspruch 25, **dadurch gekennzeichnet, dass** das Lösungsmittel ein Gemisch von entmineralisiertem Wasser und Alkohol ist.

28. Zusammensetzung gemäß Anspruch 27, **dadurch gekennzeichnet, dass** der Alkohol bis zu 80 Gew.-%, vorzugsweise 1 bis 50 Gew.-% und besonders bevorzugt 5 bis 20 Gew.-% des Gemischs ausmacht.

29. Zusammensetzung gemäß einem der vorstehenden Ansprüche, **dadurch gekennzeichnet, dass** sie keinerlei Chelatisierungsmittel des Salzes von Mn(II) und/oder Zn(II) enthält.

30. Zusammensetzung gemäß einem der vorstehenden Ansprüche, **dadurch gekennzeichnet, dass** sie in Form von zwei getrennten Komponenten vorliegt, wobei eine erste Komponente das in einem physiologisch annehmbaren Medium gelöste Katalysatorsystem umfasst und eine zweite Komponente das in einem physiologisch annehmbaren Medium gelöste Enzym und gegebenenfalls den Farbstoffvorläufer und die Aminosäure mit der Thiolgruppe umfasst.

31. Zusammensetzung gemäß einem der Ansprüche 1 bis 29, **dadurch gekennzeichnet, dass** sie in Form eines Aerosols oder eines Pumpsystems ohne Luftnachfüllung konditioniert ist.

32. Zusammensetzung gemäß Anspruch 30, **dadurch gekennzeichnet, dass** sie in einem Pumpsystem mit zwei getrennten Kammern konditioniert ist, wobei jede der Komponenten getrennt in einer der zwei Kammern enthalten ist.

33. Zusammensetzung gemäß einem der Ansprüche 1 bis 22, **dadurch gekennzeichnet, dass** sie in Form einer Tablette konditioniert ist.

34. Zusammensetzung gemäß Anspruch 33, **dadurch gekennzeichnet, dass** die Tablette eine Trägersubstanz umfasst, die Zitronensäure und/oder Weinsäure in einer substöchiometrischen Menge in Bezug auf das Alkalimetall- und/oder Erdalkalimetallhydrogencarbonat enthält.

35. Zusammensetzung gemäß einem der Ansprüche 1 bis 22, **dadurch gekennzeichnet, dass** sie in Form von zwei Tabletten konditioniert ist, wobei eine erste Tablette das Katalysatorsystem und eine Trägersubstanz umfasst und eine zweite Tablette das Enzym und gegebenenfalls den Farbstoffvorläufer und die Aminosäure mit der Thiolgruppe in einer Trägersubstanz umfasst.

36. Zusammensetzung gemäß einem der Ansprüche 33 bis 35, **dadurch gekennzeichnet, dass** die Tablette oder Tabletten Brausetabletten sind.

37. Verfahren zum Färben der Haut und/oder von Keratinfasern, **dadurch gekennzeichnet, dass** es das Auftragen einer Schicht einer Zusammensetzung gemäß einem der Ansprüche 1 bis 32 auf die Haut und/oder die Keratinfasern umfasst.

38. Verfahren gemäß Anspruch 37, **dadurch gekennzeichnet, dass** die Schicht erhalten wird, indem man einen

ersten Film aus einer Grundzusammensetzung, die das oder die Enzyme, gegebenenfalls den oder die Farbstoffvorläufer und gegebenenfalls die Aminosäure oder -säuren mit der Thiolgruppe in einem physiologisch annehmbaren Medium enthält, auf die Haut und/oder die Keratinfasern aufträgt und auf den ersten Film einen zweiten Film aus einer Katalysatorzusammensetzung, die das Katalysatorsystem in einem physiologisch annehmbaren Medium enthält, aufträgt und umgekehrt.

**39.** Verfahren gemäß Anspruch 37 oder 38, **dadurch gekennzeichnet, dass** die Filme durch Besprühen aufgetragen werden.

## Claims

**1.** A composition for colouring skin and/or keratin fibres, **characterized in that** it comprises, in a physiologically acceptable medium, an efficient amount of at least one enzyme having a propigmenting activity and an efficient amount of a catalytic system comprising a first component selected amongst salts and oxides of Mn(II) and/or Zn (II) and the mixtures thereof and a second component selected amongst alkaline hydrogen carbonates, alkaline earth hydrogen carbonates and the mixtures thereof, the proportions of the first component and the second component being such that :

$$\frac{[Mn(II)]}{[HCO_3]} \leq 1 \text{ with } [Mn(II)] \neq 0$$

$$\frac{[Zn(II)]}{[HCO_3]} \leq 1 \text{ with } [Zn(II)] \neq 0$$

$$\frac{[Mn(II)+Zn(II)]}{[HCO_3]} \leq 1 \text{ with } [Mn(II)] \text{ and } [Zn(II)] \neq 0$$

where [Mn(II)], [Zn(II)] and $[HCO_3]$ represent respectively the Mn(II), Zn(II) and $HCO_3$ molar concentrations in the composition and optionally :

- an efficient amount of at least one colouring agent precursor selected amongst the compounds comprising at least one aromatic cycle having at least two hydroxyl groups carried by two consecutive carbon atoms of the aromatic cycle and/or
- an efficient amount of at least one amino acid comprising at least one thiol group.

**2.** A composition according to claim 1, **characterized in that** the $\frac{[Mn(II)]}{[HCO_3]}$ ratio ranges from $10^{-5}$ to $10^{-1}$, preferably from $10^{-3}$ to $10^{-2}$ and more preferably is about $5.10^{-3}$.

**3.** A composition according to claim 1 or 2, **characterized in that** the $\frac{[Zn(II)]}{[HCO_3]}$ ranges from $10^{-4}$ to < 1, preferably from $10^{-3}$ to < 1 and more preferably is about $5.10^{-1}$.

**4.** A composition according to any one of the preceding claims, **characterized in that** the $\frac{[Mn(II)+Zn(II)]}{[HCO_3]}$ ratio ranges from $10^{-5}$ to $10^{-1}$, preferably from $10^{-3}$ to $10^{-2}$.

**5.** A composition according to any one of the preceding claims, **characterized in that** the Mn(II) and Zn(II) salts are selected amongst chloride, fluoride, iodide, sulfate, phosphate, nitrate, perchlorate, carboxylic acid salts and the mixtures thereof.

**6.** A composition according to claim 5, **characterized in that** the Mn(II) and/or Zn(II) salt is a chloride.

**7.** A composition according to claim 5, **characterized in that** the carboxylic acid salts are hydroxylated carboxylic acid salts.

**8.** A composition according to claim 7, **characterized in that** the hydroxylated carboxylic acid salt is a gluconate.

9. A composition according to any one of the preceding claims, **characterized in that** the hydrogen carbonate is selected amongst sodium hydrogen carbonate, potassium hydrogen carbonate, magnesium hydrogen carbonate, calcium hydrogen carbonate and the mixtures thereof.

10. A composition according to any one of the preceding claims, **characterized in that** the enzyme is selected among pyranose oxidases, glucose oxidases, glycerol oxidases, lactate oxidases, pyruvate oxidases, uricases, choline oxidases, sarcosine oxidases, bilirubin oxidases, laccases, tyrosinases, peroxydases, catalases, superoxyde dismutases and the mixtures thereof, or amongst plant and animal extracts containing the above-mentioned enzymes.

11. A composition according to claim 10, **characterized in that** the enzyme is selected amongst tyrosinases.

12. A composition according to any one of the preceding claims, **characterized in that** it comprises $5.10^{-3}$ to 5 mg, preferably from $5.10^{-2}$ to 0.5 mg enzyme per milliliter of the final composition.

13. A composition according to any one of the preceding claims, **characterized in that** the aromatic cycle comprising at least two hydroxyl groups on two consecutive carbon atoms of the colouring agent precursor is a benzene cycle or a condensed aromatic cycle.

14. A composition according to claim 13, **characterized in that** the colouring agent precursor is a compound or a mixture of compounds of formula:

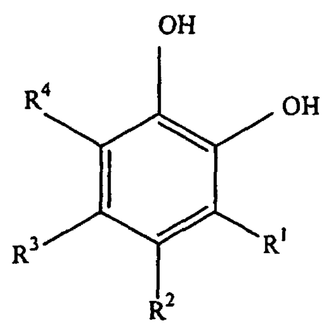

(I)

wherein the $R^1$ to $R^4$ substituents, identical or different, represent a hydrogen atom, a halogen, hydroxyl, carboxyl, alkyl carboxylate radical, an optionally substituted amino radical, a linear or branched optionally substituted alkyl radical, a linear or branched optionally substituted alcenyl radical, a optionally substituted cycloalkyl radical, an alkoxy, alkoxyalkyl, alkoxyaryl radicals, the aryl group being able to be optionally substituted, aryl, substituted aryl, an optionally substituted heterocyclic radical, a radical containing optionally one or more silicon atoms, where two of the substituents $R^1$ to $R^4$ together form a saturated or unsaturated cycle optionally containing one or more heteroatoms and optionally condensed with one ore more saturated or unsaturated cycles optionally containing one or more heteroatoms.

15. A composition according to any one of the preceding claims, **characterized in that** the colouring agent precursor is selected amongst flavanols, flavonols, anthocyanidines, anthocyanines, hydroxybenzoates, flavones, iridoids, such compounds being optionally osylated and/or in the form of oligomers, hydroxystilbenes, optionally osylated, 3,4-dihydroxyphenylalanine and the derivatives thereof, 2,3-dihydroxyphenylalanine and the derivatives thereof, 4,5-dihydroxyphenylalanine and the derivatives thereof, 4,5-dihydroxyindole and the derivatives thereof, 5,6-dihydroxyindole and the derivatives thereof, 6,7-dihydroxyindole and the derivatives thereof, 2,3-dihydroxyindole and the derivatives thereof, dihydroxycinnamates, hydroxycoumarins, hydroxyisocoumarins, hydroxycoumarones, hydroxy-isocoumarones, hydroxychalcones, hydroxychromones, anthocyans, quinones, hydroxyxanthones and the mixtures of two or more of the above-mentioned compounds.

16. A composition according to any one of the preceding claims, **characterized in that** the colouring agent precursor is selected amongst plant, fruit, citrus fruit, vegetable extracts, and the mixtures thereof.

17. A composition according to claim 16, **characterized in that** the colouring agent precursor is selected amongst tea, grape, apple, banana, potato extracts, and the mixtures thereof.

**18.** A composition according to any one of the preceding claims, **characterized in that** the colouring agent precursor is present in an amount of at least 10 micromoles per milliliter of the composition.

**19.** A composition according to any one of the preceding claims, **characterized in that** the amino acid(s) comprising at least one thiol group are selected amongst amino acids with an amine function in an $\alpha$ position relative to a carboxylic acid function.

**20.** A composition according to claim 19, **characterized in that** the amino acid(s) are selected amongst cysteine and the derivatives thereof and the glutathione and the derivatives thereof.

**21.** A composition according to any one of claims 19 to 20, **characterized in that** the molar ratio of the amino acid(s) with a SH group to the colouring agent precursor(s) ranges from 0.001 to 50, preferably from 0.01 to 5, more preferably from 0.05 to 2.5.

**22.** A composition according to any one of claims 19 to 21, **characterized in that** the colouring agent precursor is L-DOPA or a dihydroxyindole and the amino acid the L-cysteine or the glutathione

**23.** A composition according to any one of the preceding claims, **characterized in that** the physiologically acceptable medium is a solubilizing medium of the enzyme and the optional colouring agent precursor and the amino acid, preferably having a bacteriostatic property.

**24.** A composition according to claim 23, **characterized in that** the physiologically acceptable medium comprises a solvent or a mixture of solvents.

**25.** A composition according to claim 24, **characterized in that** the solvent is selected amongst demineralized water, alcohols, ethers, dimethylsulfoxide, N-methylpyrrolidone, ketones and the mixtures thereof.

**26.** A composition according to claim 25, **characterized in that** the alcohol is an alcanol or an alcanediol

**27.** A composition according to claim 25, **characterized in that** the solvent is a demineralized water/alcohol mixture.

**28.** A composition according to claim 27, **characterized in that** the alcohol represents 80% wt of the mixture, preferably 1 to 50% wt and more preferably 5 to 20% wt.

**29.** A composition according to any one of the preceding claims, **characterized in that** it is free of any chelating agent of the Mn (II) and/or Zn (II) salt.

**30.** A composition according to any one of the preceding claims, **characterized in that** it has the form of two separate components, a first component comprising the catalytic system dissolved in a physiologically acceptable medium and a second component comprising the enzyme and optionally the colouring agent precursor and the amino acid having a thiol group dissolved in a physiologically acceptable medium.

**31.** A composition according to any one of claims 1 to 29, **characterized in that** it is packed in the form of an aerosol or a pump system with no air absorption.

**32.** A composition according to claim 30, **characterized in that** it is packed in a pump system with two discrete compartments, each of the components being contained separately in one of the two compartments.

**33.** A composition according to any one of claims 1 to 22, **characterized in that** it is packed in the form of a tablet.

**34.** A composition according to claim 33, **characterized in that** the tablet comprises an excipient containing citric and/or tartaric acid in a substoichiometric amount relative to the alkaline and/or alkaline earth hydrogen carbonate.

**35.** A composition according to any one of claims 1 to 22, **characterized in that** it is packed in the form of two tablets, a first tablet comprising the catalytic system and an excipient and a second tablet comprising the enzyme and optionally the colouring agent precursor and the amino acid with an optional thiol group in an excipient.

**36.** A composition according to any one of claims 33 to 35, **characterized in that** the tablet(s) are effervescent tablets.

**37.** A method for colouring skin and/or keratin fibers, **characterized in that** it involves applying on skin and/or keratin fibres a layer of a composition according to any one of claims 1 to 32.

**38.** A method according to claim 37, **characterized in that** the layer is obtained applying on skin and/or keratin fibres a first film of a basic composition containing the enzyme(s), the colouring agent precursor(s) and the optional amino acid(s) with a thiol group in a physiologically acceptable medium, and applying on the first film a second film of a catalytic composition comprising the catalytic system in a physiologically acceptable medium or vice-versa.

**39.** A method according to claim 37 or 38, **characterized in that** the films are applied by spraying.